# EUROPEAN PATENT SPECIFICATION

(11) **EP 1 889 637 B1**
(45) Date of publication and mention of the grant of the patent: **25.12.2013**
(21) Application number: 07253194.0
(22) Date of filing: 15.08.2007
(51) Int. Cl.: A61N 5/06, A61F 11/00

(54) **Apparatus for auricular therapy**
Vorrichtung zur Ohrentherapie
Dispositif pour auriculothérapie

(30) Priority: 17.08.2006 GB 0616345
(43) Date of publication of application: 20.02.2008
(73) Proprietor: Branch, Susan Jane, Sutton Poyntz Weymouth DT3 6LQ (GB)
(72) Inventor: Branch, Susan Jane, Sutton Poyntz Weymouth DT3 6LQ (GB)
(74) Representative: Handsome I.P. Ltd

(56) References cited:
- EP-A- 1 074 275
- WO-A-00/35534
- WO-A-2006/012752
- WO-A2-98/20937
- US-A1- 2002 198 575
- US-A1- 2004 044 384
- US-A1- 2004 153 131
- US-A1- 2005 085 875
- US-A1- 2005 131 497
- US-B1- 6 459 919

## Description

This invention concerns therapeutic apparatus for treating a patient - typically but not necessarily exclusively a human patient - by the application of optical radiation to the patient's pinna, which is to say the patient's outer ear or auricle.

Auricular therapy is a development from the traditional Chinese medicine practice of acupuncture whereby specific points on the body are stimulated to restore health and wellbeing. ln auricular therapy, the whole body may be treated by stimulating acupuncture points in the patient's pinna.

Traditionally, acupuncture points have been stimulated by needles, but more recently optical stimulation has been used. In US Patent 4,535,784, for instance, Rohlicek et al describe apparatus for stimulating an acupuncture point by focusing optical radiation (visible or infra-red) upon it, and both US Patent 5,250,068 (Ideguchi et al) and US Patent 5,843,074 (Cocilovo) describe the application of light to acupuncture points by means of optical fibres. Also, as described in US Patent 4,865,035, Mori applies light to a patient's ear by means of an optical conductor cable, but not particularly to acupuncture points. WO00/35534 describes apparatus for emitting infra-red radiation to the skin covering the skull, temple regions and/or frontal lobes of an individual. The apparatus comprises two emitter units attached together by a band, so that the apparatus can be worn as a head-set.

It is to be noted that other prior devices provided a beam of radiation spread over the whole of a patient's pinna (or even more) but without any reference to stimulating acupuncture points. For instance, European Patent Application 1 086 673 (Fisher & Paykel) concerns a radiant heater with a beam focused on a patient's neck and ears (or other areas with a high concentration of arteriovenous anastomoses) so as to keep the patient warm during surgery. And US Patent 7,020,902 (Tyler) describes a headband with ear pieces holding light bulbs that radiate heat to the wearer's ears, simply for use as an ear warmer. There is no indication that such prior devices provide any substantial stimulation of acupuncture points.

In most of the prior systems for applying optical radiation to acupuncture points, the radiation is narrowly focussed onto specific acupuncture points. This was by design; as noted by Cocilovo, "When treatment of acupuncture micro-systems is preferred, such as ear acupuncture, the size of the illuminating tip is extremely important and should be as small as possible." However, this requirement of focusing stimulation on specific acupuncture points means that auricular therapy has been costly and complex.

Thus, according to the invention there is provided therapeutic apparatus for treating a patient by the application of optical radiation to the patient's pinna, the apparatus comprising a pair of earpieces connected by a band, one of said earpieces, being applicable to the patient's pinna, including a light source configured and arranged to provide a beam of optical radiation directed and extending over substantially all of the patient's pinna, the apparatus further including modulation means operable to pulse the optical radiation, characterised in that the other of said earpieces, applicable to the other of said patient's pinna, comprises an open ring with no light source.

This technique is not limited to stimulating specific acupuncture points. Since acupuncture points respond to stimulation only if they need to change, stimulating a plurality of points will bring them to homeostatic balance. It follows that stimulating the whole pinna has no detrimental effects and is as effective as stimulating individual acupuncture points. Thus, the invention provides means whereby a plurality of acupuncture points may be stimulated and brought to homeostatic balance.

Further, research carried out by the Applicant has shown that better therapeutic results are obtained from stimulating acupuncture points by radiation that is pulsed.

It should be noted here that the term 'optical radiation' as used herein is not intended to be limited to the visible range of light.

Preferably the optical radiation is pulsed at about 5 kHz. It is also preferred that the optical radiation has a rectangular waveform. It is further preferred that the optical radiation is pulsed with a duty cycle greater than 50% and more so if pulsed with a duty cycle not less than 80%.

The modulation of the optical radiation (which, it should be noted, need not be restricted to the visible range) may serve to adjust the wavelength of the radiation to tackle specific conditions. Notably, infra-red radiation, may relieve addiction, anxiety and stress; it may help patients to lose weight; and it has sedative qualities which in appropriate circumstances may reduce the need for anaesthetic. However the present invention is by no means limited to infra-red radiation, and green light has been found to have an antidepressant effect, whilst white light may be used to combat seasonal affective disorder (SAD) syndrome. The selected wavelength may be in the range 625-660 nm.

The second earpiece may be configured and arranged to allow the passage of sound, so that, for instance, the patient may comfortably listen to a television or radio programme while receiving treatment.

For improved safety, the apparatus may be powered from an electrical battery or from electrical mains by way of a step-down transformer.

The apparatus preferably comprises a timer operable to turn off the optical radiation after a predetermined period, whereupon the apparatus may deliver an audible signal.

The light source may be formed by a plurality of light emitting diodes (LEDs) for said optical radiation. Such a light source may be releasably secured to the apparatus so as to be replaceable, e.g. by a light source of another colour.

Other features of the invention will be apparent from the following description, which is made by way of example only and with reference to the accompanying schematic drawings, in which -
Figure 1 illustrates a first form of apparatus;
Figure 2 illustrates a second form of apparatus embodying the invention;
Figure 3 illustrates a third form of apparatus; and
Figure 4 outlines a system block diagram for any of the forms of apparatus shown in Figures 1 to 3.

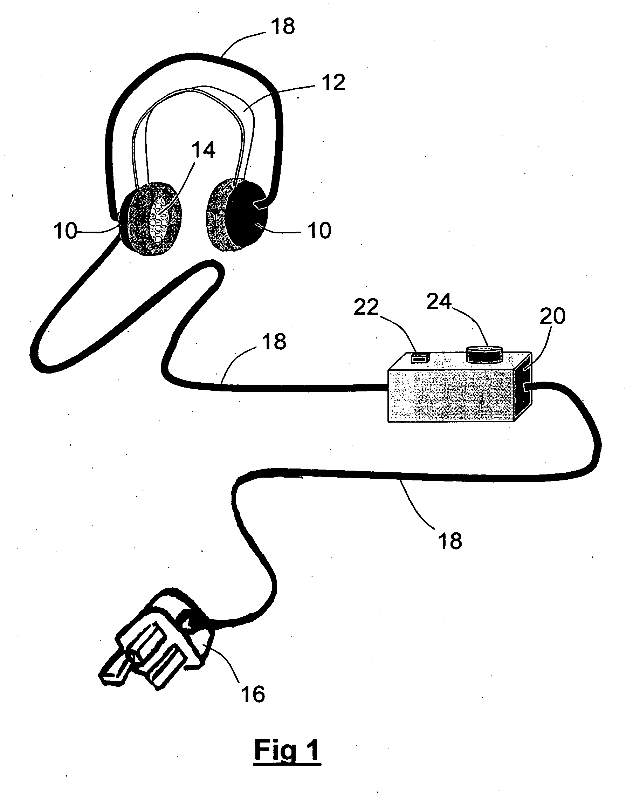

Referring first to Figure 1, this shows therapeutic apparatus comprising a pair of earpieces 10 joined by an arcuate band 12. The earpieces 10 and the band 12 are configured and arranged so that the assembly sits comfortably on a patient's head with each earpiece 10 covering one of the patient's ears. (Although not detailed in the drawings, the band 12 may be adjustable in length so as to fit the assembly more comfortably to the patient's head). Each earpiece 10 is provided with an inwardly directed light source 14, of which only one is visible in Figure 1.

Each light source 14 comprises a plurality of light emitting diodes (LEDs) powered from an electrical mains supply (not detailed) by way of a plug 16, a cable 18 and a control box 20. For safety reasons, the plug 16 includes a transformer which steps the power down to 12V dc, although the transformer may be separate from the plug 16 and may for instance be included in the control box 20.

The control box 20 comprises a switch 22 whereby the dc power to the light sources 14 is turned on. The control box 20 also comprises a rotary control 24 connected to a timing circuit within the control box for turning the power off after a predetermined period that may be varied by means of the rotary control 24, together with an audible alarm such as a beeper to indicate when such period has ended. Within the control box 20 is additional circuitry such that the supply to the light sources 14 is a 5 kHz square wave. The timing circuit and the square wave circuitry may be of any appropriate form, and it is not considered necessary to describe them in detail at this point.

The operation of the apparatus shown in Figure 1 will now be described, first in relation to a patient for whom infra-red treatment has been selected. (In the case of self-treatment, the patient himself may make the selection with reference to instructions provided with the apparatus. Otherwise the treatment may be recommended and/or administered by a trained therapist.) In the present example, infra-red treatment, with radiation in the range 625-660 nm up to, for example, 30 minutes, may be appropriate for patients suffering from stress, anxiety of addiction, or for patients wanting to lose weight, or for patients requiring sedation. For this purpose, the light sources 14 will each comprise a plurality of high intensity infra-red LEDs. The earpieces 10 are applied one to each of the patient's ears (by the therapist or by the patient himself) with the band adjusted to the patient's head, then the rotary timer 24 is set to the specified time period, and finally the apparatus is switched on by means of the switch 22. The patient can now rest, and perhaps sleep, whilst each pinna is irradiated for the specified period, at the end of which a buzzer sounds to signal that the treatment is complete. The treatment may of course be repeated as and when required and preferably under the direction of the therapist.

High intensity white LED light sources may be used for treating seasonal affective disorder, and high intensity green LEDs have an antidepressant effect. The light sources 14 may plug in to the earpieces 10 so that the colours may be changed conveniently.

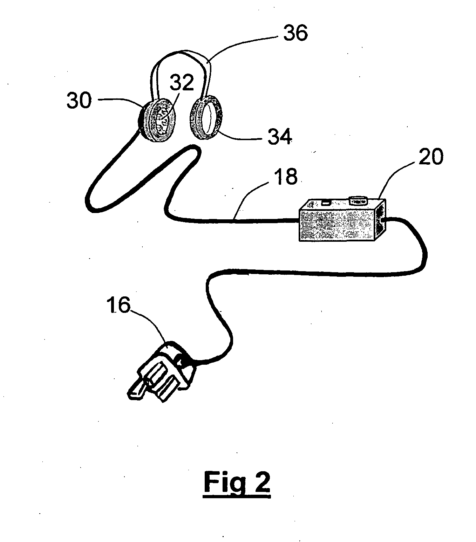

Figure 2 shows a modification of the apparatus of Figure 1. In the arrangement of Figure 2, one earpiece is substantially the same as the earpiece 10 of Figure 1 and includes a light source 32, but the other earpiece 34 simply comprises an open ring, with no light source. By this arrangement, the apparatus is comfortable for the patient and the open form of the earpiece 34 means that his hearing is unimpeded, at least on one side. Thus, patients who do not wish to simply rest during their treatment may instead listen to the radio or watch television. The cable 18 does not extend over the top of the band 36 in the apparatus of Figure 2, because power for the light source 32 is required on only one side.

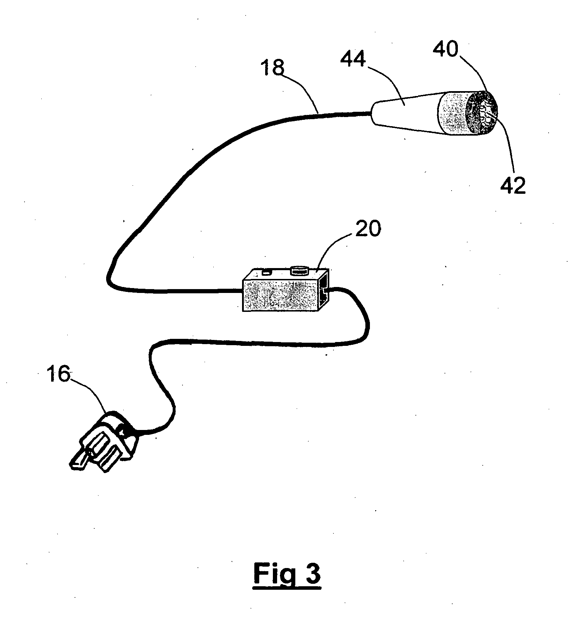

A simplified form of the apparatus is shown in Figure 3. As shown in Figure 3, an earpiece 40 with included light source 42 is mounted on the end of a barrel 44 designed to be handheld. With this form of the apparatus the earpiece 40 is simply pressed lightly upon the patient's pinna for the duration of the treatment.

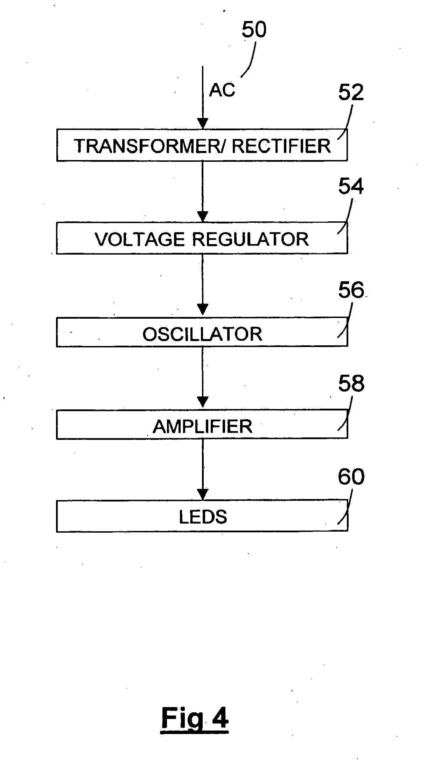

A system block diagram for the invention (which is preferably as embodied in Figure 2) is shown at Figure 4. An alternating current mains supply at 50 is fed to a transformer and rectifier 52 and then to a voltage regulator 54. The 12V output from the voltage regulator 54 powers a 5KHz square wave oscillator 56, and the output from this is then amplified by an amplifier 58 before delivery to the LED array 60.

Clinical trials of apparatus according to the invention indicate that therapeutic benefits are improved when in any treatment session the radiation is square wave pulsed with a duty cycle of 50% or above (i.e, so that the pulse duration is not less than 50% of the pulse period), and the benefits are most pronounced when the duty cycle is not less than 80%. This can be achieved by adjusting the square wave generator.

The invention has been found to be of particular benefit in treating obesity or otherwise stimulating weight loss. Causes of obesity are manifold and varied: examples are hypothyroidism, sluggish digestion, lack of exercise, overeating (which may in turn be due to poor gastric tone), low blood sugar, fluctuating blood sugar and comfort eating. With so many different possible causes, it is difficult to identify the key factors in the case of any individual patient. But the invention effectively allows the patient's body to decide how to respond, rather than the clinician having to make difficult decisions about causes and possible treatments. Thus, use of the invention with different patients wanting to lose weight has resulted in a wide range of (appropriate) patient responses including normalising of long-term constipation, causing earlier/easier satiation when the patient eats, suppressing the patient's desire to eat between meals and reducing comfort eating by making the patient feel generally happier.

Various possible modifications and adaptations may be made to the forms of the invention described. For instance, a battery may be used to power the apparatus instead of transformed mains power. Other variations will be apparent to those skilled in the art.

## Claims

1. Therapeutic apparatus for treating a patient by the application of optical radiation to the patient's pinna, the apparatus comprising a pair of earpieces (10) connected by an arcuate band (36), wherein one of said earpieces (30) applicable to the patient's pinna includes a light source (32) configured and arranged to provide a beam of optical radiation directed to the patient's pinna and extending over substantially all of the patient's pinna, the apparatus further including modulation means (20) operable to pulse the optical radiation, **characterised in that** the other of said earpieces (34) applicable to the other of said patient's pinna comprises an open ring with no light source.

2. Therapeutic apparatus as claimed in claim 1, wherein the optical radiation is pulsed at about 5 kHz.

3. Therapeutic apparatus as claimed in either one of claims 1 and 2, wherein the optical radiation has a rectangular waveform.

4. Therapeutic apparatus according to any preceding claim, wherein the optical radiation is pulsed with a duty cycle greater than 50%.

5. Therapeutic apparatus according to claim 4, wherein the optical radiation is pulsed with a duty cycle not less than 80%.

6. Therapeutic apparatus according to any preceding claim, wherein the modulation means is operable to adjust the optical radiation to a selected wavelength.

7. Therapeutic apparatus according to claim 6, wherein the selected wavelength is in the range 625-660 nm.

8. Therapeutic apparatus according to claim 6, wherein the optical radiation is of green light.

9. Therapeutic apparatus according to claim 6, wherein the optical radiation is of white light.

10. Therapeutic apparatus according to any preceding claim, wherein the earpiece containing no light source is configured and arranged to allow the passage of sound.

11. Therapeutic apparatus as claimed in any preceding claim **characterised in that** said apparatus is battery powered.

12. Therapeutic apparatus according to any one of claims 1 to 10, wherein said apparatus is powered from electrical mains by way of a step-down transformer.

13. Therapeutic apparatus according to any preceding claim, wherein said apparatus comprises a timer operable to turn off the optical radiation after a predetermined period.

14. Therapeutic apparatus according to claim 13, wherein said apparatus is configured to deliver an audible signal at the end of said predetermined period.

15. Therapeutic apparatus according to any preceding claim, wherein the light source (32) is releasably secured to the apparatus so as to be replaceable.

16. Therapeutic apparatus according to any preceding claim, wherein the light source (32) comprises a plurality of LEDs.

## Patentansprüche

1. Therapeutisches Gerät zur Behandlung eines Patienten durch Anwendung optischer Strahlung auf die Ohrmuschel des Patienten, wobei das Gerät ein Paar Ohrstücke (10) aufweist, verbunden durch ein bogenförmiges Band (36), wobei eines dieser Ohrstücke (30), anwendbar auf die Ohrmuschel des Patienten, eine Lichtquelle (32) umfasst, die ausgebildet und angeordnet ist, um einen Strahl einer optischen Strahlung zu liefern, der auf die Ohrmuschel des Patienten gerichtet ist, und sich über im wesentlichen die gesamte Ohrmuschel des Patienten erstreckt, wobei das Gerät weiterhin Modulationsmittel (20) umfasst, die bedient werden können, um die optische Strahlung zu pulsen, **dadurch gekennzeichnet, dass** das andere der Ohrstücke (34), das auf die andere Ohrmuschel des Patienten anwendbar ist, einen offenen Ring ohne Lichtquelle umfasst.

2. Therapeutisches Gerät nach Anspruch 1, bei dem die optische Strahlung mit etwa 5 kHz gepulst wird.

3. Therapeutisches Gerät nach einem der Ansprüche 1 oder 2, bei dem die optische Strahlung eine rechteckige Wellenform aufweist.

4. Therapeutisches Gerät nach einem der vorhergehenden Ansprüche, bei dem die optische Strahlung mit einem Arbeitszyklus größer als 50 % gepulst wird.

5. Therapeutisches Gerät nach Anspruch 4, bei dem die optische Strahlung mit einem Arbeitszyklus von nicht weniger als 80 % gepulst wird.

6. Therapeutisches Gerät nach einem der vorhergehenden Ansprüche, bei dem die Modulationsmittel so bedienbar sind, dass die optische Strahlung auf eine ausgewählte Wellenlänge eingestellt wird.

7. Therapeutisches Gerät nach Anspruch 6, bei dem die ausgewählte Wellenlänge in dem Bereich 625-660 nm liegt.

8. Therapeutisches Gerät nach Anspruch 6, bei dem die optische Strahlung grünes Licht ist.

9. Therapeutisches Gerät nach Anspruch 6, bei dem die optische Strahlung weißes Licht ist.

10. Therapeutisches Gerät nach einem der vorhergehenden Ansprüche, bei dem das Ohrstück, welches keine Lichtquelle enthält, ausgebildet und angeordnet ist, den Durchgang von Schall zu ermöglichen.

11. Therapeutisches Gerät nach einem der vorhergehenden Ansprüche, **dadurch gekennzeichnet, dass** das Gerät von einer Batterie versorgt wird.

12. Therapeutisches Gerät nach einem der Ansprüche 1 bis 10, bei dem das Gerät über das elektrische Netz versorgt wird mittels eines Abwärts-Transformators.

13. Therapeutisches Gerät nach einem der vorhergehenden Ansprüche, bei dem das Gerät eine Zeituhr umfasst, die bedienbar ist, um die optische Strahlung nach einem vorgegebenen Zeitraum auszuschalten.

14. Therapeutisches Gerät nach Anspruch 13, bei dem das Gerät ausgebildet ist, um
ein hörbares Signal am Ende des vorgegebenen Zeitraums zu liefern.

15. Therapeutisches Gerät nach einem der vorhergehenden Ansprüche, bei dem die Lichtquelle (32) lösbar an dem Gerät festgelegt ist, so dass sie austauschbar ist.

16. Therapeutisches Gerät nach einem der vorhergehenden Ansprüche, bei dem die Lichtquelle (32) eine Mehrzahl von LEDs umfasst.

## Revendications

1. Appareil thérapeutique pour traiter un patient par l'application d'un rayonnement optique au pavillon de l'oreille du patient, l'appareil comprenant une paire d'écouteurs (10) reliés par une bande arquée (36), l'un desdits écouteurs (30), apte à être appliqué au pavillon de l'oreille du patient, comprenant une source de lumière (32) configurée et agencée pour fournir un faisceau de rayonnement optique dirigé vers le pavillon de l'oreille du patient et s'étendant sur sensiblement tout le pavillon de l'oreille du patient, l'appareil comprenant en outre des moyens de modulation (20) actionnables pour pulser le rayonnement optique, **caractérisé par le fait que** l'autre desdits écouteurs (34), apte à être appliqué à l'autre pavillon de l'oreille dudit patient, comprend un anneau ouvert sans source de lumière.

2. Appareil thérapeutique selon la revendication 1, dans lequel le rayonnement optique est pulsé à environ 5 kHz.

3. Appareil thérapeutique selon l'une ou l'autre des revendications 1 et 2, dans lequel le rayonnement optique a une forme d'onde rectangulaire.

4. Appareil thérapeutique selon l'une quelconque des revendications précédentes, dans lequel le rayonnement optique est pulsé avec un facteur d'utilisation supérieur à 50 %.

5. Appareil thérapeutique selon la revendication 4, dans lequel le rayonnement optique est pulsé avec un facteur d'utilisation non inférieur à 80 %.

6. Appareil thérapeutique selon l'une quelconque des revendications précédentes, dans lequel les moyens de modulation sont actionnables pour régler le rayonnement optique à une longueur d'onde choisie.

7. Appareil thérapeutique selon la revendication 6, dans lequel la longueur d'onde choisie se situe dans la plage de 625-660 nm.

8. Appareil thérapeutique selon la revendication 6, dans lequel le rayonnement optique est de lumière verte.

9. Appareil thérapeutique selon la revendication 6, dans lequel le rayonnement optique est de lumière blanche.

10. Appareil thérapeutique selon l'une quelconque des revendications précédentes, dans lequel l'écouteur ne contenant pas de source de lumière est configuré et agencé pour autoriser le passage du son.

11. Appareil thérapeutique selon l'une quelconque des revendications précédentes, **caractérisé par le fait que** ledit appareil est alimenté par batterie.

12. Appareil thérapeutique selon l'une quelconque des revendications 1 à 10, dans lequel ledit appareil est alimenté depuis le réseau électrique au moyen d'un transformateur abaisseur.

13. Appareil thérapeutique selon l'une quelconque des revendications précédentes, dans lequel ledit appareil comprend un minuteur actionnable pour arrêter le rayonnement optique après une période prédéterminée.

14. Appareil thérapeutique selon la revendication 13, dans lequel ledit appareil est configuré pour fournir un signal audible à la fin de ladite période prédéterminée.

15. Appareil thérapeutique selon l'une quelconque des revendications précédentes, dans lequel la source de lumière (32) est fixée de manière libérable à l'appareil de façon à être remplaçable.

16. Appareil thérapeutique selon l'une quelconque des revendications précédentes, dans lequel la source de lumière (32) comprend une pluralité de DEL.
